# EUROPEAN PATENT SPECIFICATION

(11) **EP 3 683 302 B1**
(45) Date of publication and mention of the grant of the patent: **21.02.2024**
(21) Application number: 18857324.0
(22) Date of filing: 14.09.2018
(51) Int. Cl.: C12N 1/18, C12R 1/865, C12P 7/10, C12G 1/022, A21D 8/04

(54) **STRAIN OF SACCHAROMYCES CEREVISIAE AND USE THEREOF FOR MAKING ALCOHOLIC PRODUCTS**
SACCHAROMYCES CEREVISIAE-STAMM UND SEINE VERWENDUNG ZUR HERSTELLUNG ALKOHOLISCHER PRODUKTE
SOUCHE DE SACCHAROMYCES CEREVISIAE ET UTILISATION DE CELLE-CI POUR LA PRODUCTION DE PRODUITS CONTENANT DE L'ALCOOL

(30) Priority: 14.09.2017 ES 201731116
(43) Date of publication of application: 22.07.2020
(73) Proprietor: Tomsa Destil, S.L., 28042 Madrid (ES)
(72) Inventor: VILLENA REYES, Miguel, 28050 Madrid (ES); AGUADO RAMOS, Manuel, 14005 Córdoba (ES); NÚÑEZ GUTIÉRREZ, Yolanda, 28047 Madrid (ES); MAGANTO BURGOS, Elena, 28701 San Sebastián De Los Reyes (Madrid) (ES); HERNÁNDEZ MATEO, Daniel, 28002 Madrid (ES)
(74) Representative: González López-Menchero, Álvaro Luis
(86) International application number: PCT/ES2018/070602
(87) International publication number: WO 2019/053316

(56) References cited:
- EP-A1- 0 798 382
- WO-A1-00/04190
- WO-A1-2010/064759
- WO-A1-2015/035214
- WO-A1-2017/090055
- ES-A1- 2 350 223
- ES-A1- 2 350 431
- BAJAJ B. K. ET AL: "Construction of killer industrial yeast Saccharomyces cerevisiae HAU-1 and its fermentation performance", BRAZILIAN JOURNAL OF MICROBIOLOGY, vol. 41, no. 2, 1 April 2010 (2010-04-01), pages 477-485, XP055131563, ISSN: 1517-8382, DOI: 10.1590/S1517-83822010000200030
- SEKI T. ET AL: "Genetic Construction of Yeast Strains for high Ethanol Production", BIOTECHNOLOGY LETTERS, vol. 5, no. 5, 1 May 1983 (1983-05-01), pages 351-356, XP002696474, ISSN: 0141-5492, DOI: 10.1007/BF01141137
- LUCCA M. E. ET AL: "Characterisation of osmotolerant hybrids obtained by fusion between protoplasts of Saccharomyces cerevisiae and heat treated protoplasts of Torulaspora delbrueckii", BIOTECHNOLOGY LETTERS, vol. 21, no. 4, 1 April 1999 (1999-04-01), pages 343-348, XP002546977, ISSN: 0141-5492, DOI: 10.1023/A:1005488808895
- SEKI, T. et al.: "Genetic construction of yeast strains for high ethanol production", Biotechnology Letters, vol. 5, no. 5, 1983, pages 351-356, XP002696474, DOI: 10.1007/BF01141137
- BUESHER, W. A. et al.: "High alcohol wine production from grape juice concentrates", American journal of enology and viticulture : AJEV, vol. 52, no. 4 2001, pages 345-351, XP009519907, ISSN: 0002-9254 Retrieved from the Internet: URL:https://www.ajevonline.org/content/52/ 4/345
- MALACRINO P. et al.: "The vinification of partially dried grapes: a comparative fermentation study of Saccharomyces cerevisiae strains under high sugar stress", Letters in Applied microbiology, vol. 40, no. 6, 2005, pages 466-472, XP055582824, DOI: 10.1111/j.1472-765X.2005.01713.x
- BERTOLONI, M.C. et al.: "New strains for alcoholic fermentation at higher sugar concentration", Biotechnology Letters, vol. 13, no. 3, 1991, pages 197-702, XP055582827,
- GUIJO, S. et al.: "Fermentative features of vinification and maturation yeasts isolated in the Montilla-Moriles region of Southern Spain", Food Microbiology, vol. 3, no. 2, 1986, pages 133-142, XP055582829, ISSN: 0740-0020, DOI: Fermentative features of vinification and maturation yeasts isolated in the Montilla-Moriles region of Southern Spain

## Description

### Field of the invention

The present invention belongs to the technical field of alcoholic fermentations, such as the production of wine, beer, alcohol, etc., for human consumption. More specifically, the present invention relates to a new osmo-ethanol-tolerant strain of yeast of the species *Saccharomyces cerevisiae,* and its use for producing ethanol or preparing alcoholic products. The strain described is also useful for the production of biomass.

### Background of the invention

Osmotic stress has a remarkable influence on the cell physiology of *Saccharomyces cerevisiae* strains, which is shown in the dynamics of cell wall changes, in the alteration of ion homeostasis, in metabolic adjustments, in cell cycle arrest, as well as a very remarkable effect on gene expression. Therefore, osmotic stress is a factor that negatively affects both cell proliferation and fermentation capacity of *Saccharomyces cerevisiae* strains and, finally, alcohol production at the industrial level.

Therefore, there is a constant need to develop new strains of *Saccharomyces cerevisiae* with improved characteristics and in particular with high osmo-ethanol-tolerance.

Some *S. cerevisiae* strains with improved characteristics have already been described in the literature, e.g. in ES2116922, ES2524704 or ES2406412. Bajaj et al., Brazilian Journal of Microbiology (2010) 41 :477-485 discloses a S. *cerevisiae* strain obtained by protoplast fusion of *S. cerevisiae* HAU-1 with *S. cerevisiae* MTCC 475, thereby obtaining a yeast strain having ethanol resistance, osmotolerance and high ethanol production, and killer activity which is stably maintained during hostile conditions of ethanol fermentations in dextrose or molasses and even during biomass recycling.

The authors of the present invention have obtained a new hybrid strain of yeast of the species *Saccharomyces cerevisiae* by the technique of protoplast fusion from the highly osmo-tolerant strain *S*. *cerevisiae NCYC73* and another strain *S. cerevisiae.* This technique produces a chromosomal exchange mainly of chromosomes of lower molecular weight, resulting in a new yeast strain in which characteristics and capabilities of both parental strains coexist.

In particular, the strain obtained by the authors has a high osmo-ethanol-tolerant capacity and a capacity to produce 16° G.L or higher.

### Brief description of the invention

The main aspect of the invention is an osmo-ethanol-tolerant strain of yeast of the species *Saccharomyces cerevisiae,* (strain of the invention), which was deposited on July 4^{th}, 2017 in the Spanish Type Culture Collection (CECT) (Burjassot, Valencia, Spain) and which was assigned the access number CECT 13152.

In another relevant aspect, the invention relates to the use of the strain of the invention for the production of ethanol or alcoholic products by fermentation using different substrates.

Another aspect of the invention relates to the use of yeast for the production of biomass.

A final aspect of the invention relates to a microbiological composition comprising a strain of the invention and optionally at least one additional element that may benefit the production of alcohols or alcoholic products.

### Brief description of the figures

**Figure 1****:** shows an outline of the cell wall removal in the parental yeast strains.
**Figure 2****:** representation of the protoplast fusion of parental cells, with protoplast A being a highly osmo-tolerant yeast and protoplast B being a yeast used in industrial fermentation.

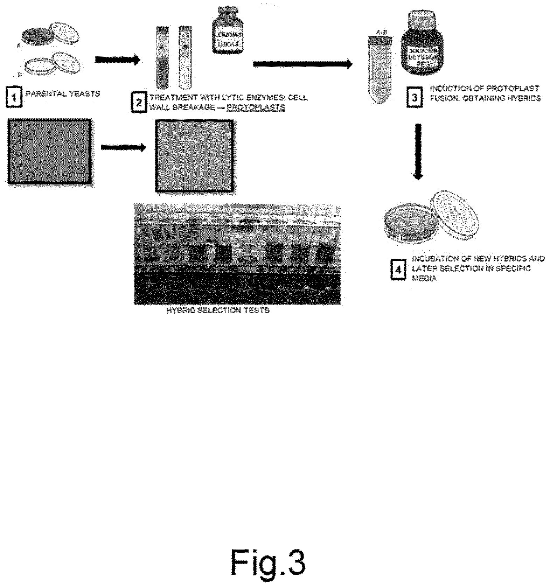

**Figure 3****:** a scheme of the production of the strain of the invention by means of protoplast fusion technique is shown, involving the sequence of treatment with lytic enzymes of parental yeasts, induction of protoplast fusion, incubation of new hybrids and selection in specific media.

### Detailed description of the invention

The present invention relates in its main aspect to a strain of the species *Saccharomyces cerevisiae* deposited in the Spanish Type Culture Collection (CECT) with access number CECT 13152.

The main characteristic of this strain is that it is highly osmo-ethanol-tolerant, which makes it particularly suitable for the production of ethanol by means of alcoholic fermentation from different substrates, as well as for the production of alcoholic products such as alcoholic beverages (wine, beer, cava or cider). In fact, the strain of the present invention is capable of producing ethanol or alcoholic products by fermentation at 16° G.L. or higher.

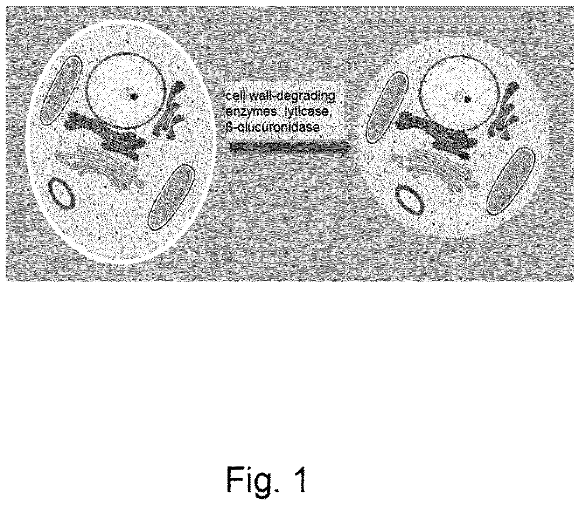

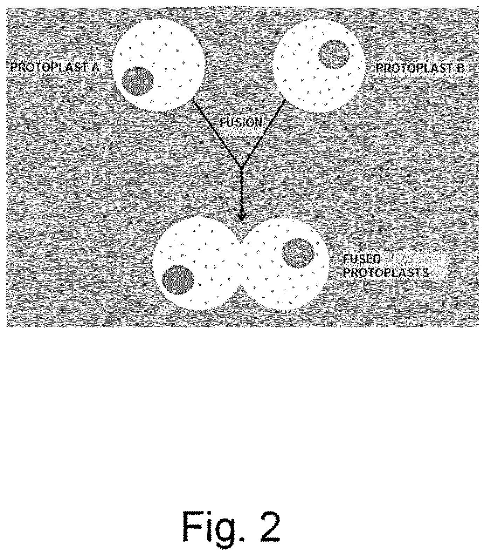

The strain of the invention has been obtained by the technique of protoplast fusion which allows the genetic exchange of parental cells through a previous stage of digestion of their cell walls and a subsequent stage of membrane fusion. Figures 1 and 2 show a graphic representation of both stages.

Therefore the strain of the invention is a hybrid strain resulting from the fusion of a highly osmo-tolerant strain of *S.cerevisiae,* the NCYC73 strain and another strain of *S.cerevisiae* used in industrial fermentation. The strain obtained has characteristics and capacities of both parental strains and particularly shows a high osmo-ethanol-tolerance.

The strain of the invention can be transported in liquid, fresh paste, active dry or instant dry format.

The strain of the invention has an ovoid morphology and a matt beige color. The metabolic characteristics observed are similar to those of other industrial strains of S. *cerevisiae* and in particular to those of parental strains from which it is derived. The rapid growth and rapid metabolization of fermentable sugars, as well as the high osmo-ethanol-tolerance of the strain of the invention make it particularly suitable for industrial use and particularly in the production of bioethanol or in the production of alcoholic beverages.

Another aspect of the invention relates to the use or application of the strain of the invention. On the one hand, the strain of the invention is useful in the production of ethanol or the manufacture of alcoholic products by fermentation.

In a particular embodiment, the strain of the invention is useful for the production of bioethanol from plant residues and lignocellulosic residues. Plant residues or lignocellulosic residues can be the biodegradable fraction of products, residues and remains of origin coming from agriculture such as crop residues, rich in fermentable sugars, such as sugar cane; starch biomass, for example, wheat grains or straw; or corn or corn straw or corn grain or corn fiber; or barley grains or straw; or grains or straw of sorghum, rice, grass, branches, etc. Plant residues and lignocellulosic residues can also come from forest industries such as woodworking.

As regards the application for the production of alcoholic products by fermentation, the strain of the invention is particularly suitable for the production of alcoholic beverages and more particularly of beverages such as wine, beer, champagne, cider or distilled beverages.

The use of the strain of the invention makes it possible to obtain by alcoholic fermentation ethanol or alcoholic products at 16° G.L. or higher.

Likewise, another use of the strain of the invention is intended for baking or for the production of bread or pastry and confectionery products.

Another use of the strain of the invention is intended for the production of biomass as a means of transforming fermentable plant or animal residues (whey) of low added value into biomass with a high protein value.

In one particular relationship, the biomass obtained is applied as a source of animal feed, particularly for livestock.

A final aspect of the invention relates to a microbiological composition comprising the strain of the invention and, optionally, at least one additional element that may benefit the production of alcohols or alcoholic products and/or the fermentation process.

In a particular embodiment of the invention the composition can be presented in the form of a diluted aqueous, cream, pressed, dried or freeze-dried composition.

Specific examples of embodiments of the invention that illustrate the invention are detailed below.

### Examples

### Example 1: Obtaining the mutant Saccharomyces cerevisiae of the invention (CECT 13152).

In order to obtain the *Saccharomyces cerevisiae* strain deposited in the Spanish Type Culture Collection (CECT) with the access number CECT 13152, protoplast fusion methods similar to those described in *"*Induced Fusion of Fungal Protoplasts following Treatment with Polyethylene Glycol" were used. Journal of General Microbiology (1976), 92, 4 I 3- 4 17. J. Anne and J. F. Peberdy or *"*Fusion of protoplasts of Bacillus megaterium. Proc. Nati. Acad. Sci. USA Vol. 73, No. 6, pp. 2147-2150, June 1976 Microbiology. Katalin Fodor and Lajos Alfoldi.

The following are the steps that were carried out to obtain the deposited mutant with CECT number 13152

### Growth of parental strains

The parental strains were grown in glycerol agar plates, to make sure they were breathing properly. On the one hand, the NCYC73 strain of *S.cerevisiae* with a highly osmo-tolerant capacity and, on the other hand, other *S.cerevisiae* strains for industrial use were used as the parental strains.

Once grown on these plates, 1, 2 or 3 loops of these yeasts were inoculated into a liquid medium, the composition of which depended on the osmo-tolerance condition of the yeast.

Osmo-tolerant yeasts, in a medium containing glucose, produce a harder wall, which is more difficult to degrade in the lysis phase. For this reason, galactose was used, so that the resulting wall was looser. Once the media were inoculated, they were kept at 25° to 35°C and at 50 to 500 rpm, for one night.

The incubation time depends on the yeast growth curve, so they were removed from the shaker at the end of the exponential phase and before entering the stationary phase.

### Re-suspension of yeast in pre-treatment buffer.

For the fusion, the first thing to do is to get a yeast cream pellet. To do this, 10 to 50 ml of the culture medium was taken and centrifuged at 500 to 5000 rpm for 1 to 10 minutes and then the supernatant was removed until leaving 1 to 12 ml. It was re-suspended and centrifuged again at 1000-5000 rpm for 1-10 minutes.

### Preparation of the pre-treatment buffer.

To the pre-treatment buffer Tris-EDTA pH between 6 and 9 previously prepared and autoclaved, β-mercaptoethanol was added in the following proportion: between 4 and 11 ml of Tris-EDTA buffer + 100 - 400µl of β-mercaptoethanol, stirring well. The β-mercaptoethanol was added to a 15ml falcon containing between 4 and 11 ml of buffer. The buffer had the appropriate molarity for the osmo-tolerance condition of the yeast we are using. As a rule, if they were not osmo-tolerant, a buffer with sorbitol between 0.2 and 1 M or KCI between 0.2 and 1 M was used. On the contrary, for osmo-tolerant ones, the buffer was used with a molarity between 0.1 and 1.1 M with sorbitol or between 0.5 and 2 M with KCl.

### Yeast pre-treatment

The supernatant of the yeast was removed after the last centrifugation. Between 4 and 12 ml of pre-treatment buffer containing β-mercaptoethanol was added. It was stirred well using a vortex and the mixture was poured into the shaker at 30-45°C and at 50-200 rpm for 10 to 50 minutes. It was kept for additional 5 to 15 minutes at the revolutions indicated above. The mixture was centrifuged again at 1000-5000 rpm for 1-20 minutes. At this level the wall was already beginning to degrade.

### Preparation of the lysis buffer

To prepare the lysis buffer, lytic enzymes were incorporated into a phosphate-citrate buffer (with sorbitol) pH between 4 and 7.

Just before incorporating the lysis buffer on the yeast, it was filtered with a sterile filter between 0.1 and 1µm. One or two filters were used to completely filter between 1 and 10 ml of buffer.

### Completion of cell wall lysis

Almost all of the supernatant was removed from the falcon, and between 1 and 10 ml of the sterile lysis buffer was added. Then the yeast was re-suspended. The mixture was incubated at 30 to 40°C again and stirred at 50 to 500 rpm for 10 to 50 minutes. After 10 to 50 minutes, the spheroplasts began to appear in the suspension.

To determine at what point the lysis had been completed, water testing was performed based on the fragility of unwalled cells faced to osmotic variations in the extracellular medium. To carry it out, between 5 and 75 µl of the yeast solution was introduced in 0.5 and 6 ml of milliQ water. When the solution was translucent, it meant that yeasts had not yet lost their walls, while when the solution was transparent, with small off-white lumps, it meant that the yeasts had lost their walls, and when being introduced into a hypotonic medium, they did not resist the change.

If we have incorrectly chosen the molarity of the buffer, it may be that the cells do not lose the wall correctly, that they suffer a modification in their shape, or in their size (due to water loss or absorption). In these cases, the yeast cannot be used for melting. Osmo-tolerant yeasts take longer to lyse their cell wall.

While the solution remained translucent, the yeast was put back into the shaker and the test was repeated after 10 to 60 minutes. That is, until the cells broke down in water. To compare the response of the yeast in water with a negative control, 5 to 80 µl of yeast was introduced into 0.5 to 6 ml of buffer.

The formation of the spheroplasts was also visually checked by microscopic observation.

### Washing of cells and measurement of cell population

Once the spheroplasts were formed, the cells were washed for fusion. This was done using Tris-HCl pH 6-9 wash buffer with sorbitol (at the corresponding molarity). Thus, once the falcon was removed from the shaker, between 1 and 15 ml of the wash buffer was added and, by stirring, it was centrifuged at 500 to 5000 rpm for 1 to 15 minutes. The supernatant was removed and the procedure was repeated. This procedure was performed a total of one to six times.

The last time, after removing the supernatant, 1-6 ml of the wash buffer was added and carefully re-suspended. Then 1 to 20 µl of the yeast suspension were taken and dissolved into 450 to 1000 µl of milliQ water. A small amount of this dilution was pipetted into the Neubauer chamber (40X lens). The millions of cells were counted, and depending on the population, the ml to be added from the suspension was determined to collect between 100 and 2000 million cells. The important thing was that there should be the same amount of both yeast strains to fuse.

### Incubation of the yeast in the melting solution

The amount of suspension of spheroplasts from the first yeast that was calculated according to the population was introduced into an eppendorf. It was then centrifuged at 1000 to 7000 rpm for 1 to 50 seconds. Once this was done, the supernatant was removed and the amount of suspension from the second yeast was added. It was then centrifuged again at 1000 to 7000 rpm for the same time. The supernatant was removed again and 0.1 to 5 ml of the fusion solution was added. The PEG makes the solution very dense and difficult to aspirate. It was re-suspended and incubated in an oven at 27 to 40°C for 10 to 50 minutes.

### Growth of hybrids in solid media

After 10 to 50 minutes of fusion, 10 to 75 ml of 1.5% E-type agar solution were taken which were autoclaved and warmed to 40 and 70°C, and 10 to 65 ml of 0.5 to 2M CaCl₂ were taken which were sterilized with a 0.1 to 0.5µm filter and also warmed to 40 to 70°C and mixed well. Between 50 and 300µl of the fusion solution was added to the mixture with the yeasts and stirred to disperse the hybrids throughout the agar.

Finally, this mixture was poured over the OSY plates that were incubated at 20 to 40°C for 1 to 5 days.

### Later phases.

The colonies that grew in the OSY medium, were taken and seeded in the YPD medium, and from there they were moved to glycerol agar between 1 and 5%. In the YPD medium they were seeded by taking all the large colonies, forming 4 or 5 striae on the plate. From the glycerol agar a reserve tube was prepared using 10 to 50% glycerol medium.

From the different strains isolated, the strain of the invention deposited with access number CECT 13152 was selected.

### Example 2: Growth kinetics of the mutant Saccharomyces cerevisiae CECT 13152.

In order to study the growth kinetics, samples of both the parental strains and the new mutant strain CECT 13152 were inoculated into YPD broth. A material balance was made based on the nutritional requirements of the yeast by formulating three media, based on agro-industrial residues, tubers, cereal hydrolysates and fruit wastes. The pH of the samples was regulated until reaching the range of 4.5-5.0. After mixing and homogenizing, the media were autoclaved at 121°C for 15 minutes.

The culture media were placed in glass balls of 2L capacity; the aeration mechanism was connected to this system through low power pumps together with diffuser stones, these were disinfected and introduced in the respective culture media. Finally, the harvested yeasts were inoculated. The systems were covered with cotton to avoid external contamination.

Measurements were made every hour, for which a 10 mL sample was taken from each culture. Growth was quantified using Neubauer chamber with an optical microscope at 400X magnification; °Brix and pH were controlled during biomass production. Modified versions of the Logistic and Gompertz models proposed by Zwietering et al, (1990) were used.

This made it possible to determine the growth kinetics through maximum growth (Yₘₐₓ), lag or adaptation phase (λ), specific growth rate (µₘₐₓ). The generation time (G) was calculated: G = log 2/ µₘₐₓ.

The results showed that the generation time of *Saccharomyces cerevisiae* CECT 13152 for both parental strains increased by at least 20%, without increasing the duration of the latency period.

### Example 3: In vitro evaluation of ethanol production capacity and osmo-tolerance of the mutant Saccharomyces cerevisiae CECT 13152.

A comparative test of alcohol production capacity was carried out between a commercial strain of *Saccharomyces cerevisiae* and the CECT 13152 strain under different conditions and on different substrates.

Table 1 below summarizes the conditions and parameters that were measured before and after the fermentation test using brown sugar as a carbon source:

**TABLE 1**

| **CO DE** | **INITIAL PARAMETERS** | | | | | | | **FINAL PARAMETERS** | | | | | | |
|---|---|---|---|---|---|---|---|---|---|---|---|---|---|---|
| | **INITI AL SUG ARS (g/10 0mL)** | **LEV** | **BUF FER** | **DEN SITY (g/c m³)** | **POPUL ATION (mill/mL )** | **P H** | **ACI DITY (% aceti c acid)** | **°GL DISTIL LATE** | **FERMEN TATION TIME** | **SUG ARS (%)** | **P (g/c m3)** | **POPUL ATION (mill/mL )** | **P H** | **ACI DITY (% aceti c acid)** |
| 1 | 20 | Comm ercial | NOT | 1,080 | 73 | 4, 40 | <0,0 1 | 5,8 | 90 | 11,5 | 1,03 6 | 106 | 2, 9 | 0,25 |
| 2 | 17 | | | 1,070 | 80 | 4, 34 | <0,0 1 | 5,6 | 90 | 8,48 | 1,02 5 | 133 | 3, 3 | 0,21 |
| 3 | 20 | CECT 13152 | | 1,080 | 75 | 4, 40 | <0,0 1 | 7,2 | 72 | 8,15 | 1,02 4 | 111 | 3, 16 | 0,25 |
| 4 | 17 | | | 1,070 | 70 | 4, 34 | <0,0 1 | 6,6 | 72 | - | 1,01 6 | 101 | 3, 13 | 0,22 |
| 5 | 20 | Comm ercial | YES | 1,086 | 90 | 4, 48 | 0,33 | 7,9 | 90 | - | 1,02 5 | 155 | 3, 81 | 0,59 |
| 6 | 17 | | | 1,075 | 145 | 4, 51 | 0,34 | 7,7 | 90 | 4,69 | 1,01 6 | 161 | 3, 75 | 0,60 |
| 7 | 20 | CECT 13152 | | 1,086 | 128 | 4, 48 | 0,33 | 11,0 | 72 | - | 1,00 4 | 110 | 4, 07 | 0,53 |
| 8 | 17 | | | 1,075 | 120 | 4, 51 | 0,34 | 10,2 | 72 | 0,18 | 0,99 6 | 120 | 4, 03 | 0,55 |

The first thing that can be observed is that the strain of the invention has a high osmo-tolerance to sugars and is capable of growing and metabolizing sugars at a high concentration of up to at least 28 g/100ml.

On the other hand, as it can be seen the degree of distilled alcohol is substantially higher in the case of the CECT13152 strain than in the case of the commercial strain under all the conditions tested, which shows that the strain is suitable for industrial use.

Table 2 also summarizes some of the parameters that demonstrate the higher fermentation and alcohol production capacity of the strain of the invention:

**Table 2**

| **CODE** | **1** | **2** | **3** | **4** | **5** | **6** | **7** | **8** | **UNITS** |
|---|---|---|---|---|---|---|---|---|---|
| **η _{THEORETICAL}** | 0.5 11 | 0.5 11 | 0.51 1 | 0.51 1 | 0.51 1 | 0.51 1 | 0.51 1 | 0.51 1 | g EtOH/g glucose |
| **DISTILLED ALCOHOL CONTENT** | 5.8 | 5.6 | 7.2 | 6.6 | 7.9 | 7.7 | 11 | 10.2 | °GL |
| | 45. 80 | 44. 22 | 56.8 5 | 52.1 1 | 62.3 8 | 60.8 0 | 86.8 6 | 80.5 4 | g Pure EtOH/L |
| **FERMENT SUGAR OVER ALCOHOL** | 89. 60 | 86. 51 | 111. 23 | 101. 96 | 122. 04 | 118. 95 | 169. 94 | 157. 58 | g fermentable sugars/L |
| **FERMENTABLE SUGARS MEASURED HPLC** | 200 | 170 | 200 | 170 | 200 | 170 | 200 | 170 | g/L |
| **SUBSTRATE CONCENTRATION** | 200 | 170 | 200 | 170 | 200 | 170 | 200 | 170 | g/L |

| **% FERMENTABLE SUGARS IN THE ORIGINAL SUBSTRATE (WITHOUT DILUTION)** | | | | | | | | | |
|---|---|---|---|---|---|---|---|---|---|
| **THEORETICAL (WITH RESPECT TO °GL OBTAINED)** | 45 | 51 | 56 | 60 | 61 | 70 | 85 | 93 | % |
| **ACTUAL (WITH RESPECT TO MEASURED SUGARS)** | 100 | 100 | 100 | 100 | 100 | 100 | 100 | 100 | % |
| **η _{MAXIMUM ETHANOL}** | 290 | 329 | 360 | 388 | 395 | 453 | 550 | 600 | L.A.P./ Tm substrate |

Similarly, a comparative test of the alcohol production capacity between a commercial strain of *Saccharomyces cerevisiae* and the CECT 13152 strain was carried out for different percentages of molasses as substrate.

The following Tables 3A and 3B detail the codes of the different tests carried out which identify both the percentage of molasses used and the total sugars (numerical code) as well as the strain used (alphabetical code):

**Table 3A and 3B**

| **Commercial** | |
|---|---|
| **1A** | 20% MOLASSES (25% TOTAL SUGARS) |
| **2A** | 20% MOLASSES (20% TOTAL SUGARS ) |
| **3A** | 25% MOLASSES (17.2% TOTAL SUGARS ) |
| **4A** | 25% MOLASSES (25% TOTAL SUGARS) |
| **5A** | 25% MOLASSES (20% TOTAL SUGARS ) |

| **CECT** 13152 | |
|---|---|
| **1b** | 20% MOLASSES (25% TOTAL SUGARS) |
| **2b** | 20% MOLASSES (20% TOTAL SUGARS ) |
| **3b** | 25% MOLASSES (17.2% TOTAL SUGARS ) |
| **4b** | 25% MOLASSES (25% TOTAL SUGARS) |
| **5b** | 25% MOLASSES (20% TOTAL SUGARS ) |

Table 4 below summarizes the parameters that were measured before and after the fermentation test using molasses as a carbon source:

**Table 4**

| **COD E** | **INITIAL PARAMETERS** | | | | | **FINAL PARAMETERS** | | | | | | |
|---|---|---|---|---|---|---|---|---|---|---|---|---|
| | **P (g/cm 3)** | **POPULATI ON (mill/mL)** | **BRI X** | **PH** | **ACIDI TY (% acetic acid)** | **°GL DISTILL ATE** | **FERMENTAT ION TIME** | **ρ (g/cm 3)** | **POPULATI ON (mill/mL)** | **PH** | **ACIDI TY (% acetic acid)** | **BRI X** |
| 1A | 1,134 | 141 | 32 | 4,4 5 | 0,56 | 13.0 | 120 | 1,036 | 188 | 4,6 4 | 0,72 | 17. 6 |
| 1B | | 105 | | | | 15.1 | 76 | 1.025 | 118 | 4,6 1 | 0,74 | 17. 4 |
| 2A | 1,110 | 101 | 27 | 4,5 3 | 0,53 | 10,4 | 120 | 1,027 | 170 | 4,4 3 | 0,82 | 14, 4 |
| 2B | | 118 | | | | 12 | 42 | 1.020 | 183 | 4,6 0 | 0,77 | 14, 0 |
| 3A | 1,108 | 116 | 26 | 4,4 6 | 0,71 | 9,5 | 120 | 1,036 | 191 | 4,6 0 | 0,89 | 15, 8 |
| 3B | | 120 | | | | 9,7 | 50 | 1,040 | 200 | 4,6 1 | 0,92 | 15, 4 |
| 4A | 1,138 | 71 | 33 | 4,4 5 | 0,70 | 3,8 | 126 | 1,109 | 123 | 4,4 9 | 0,88 | 28, 6 |
| 4B | | 95 | | | | 9.2 | 91 | 1,067 | 176 | 4,4 5 | 0,93 | 22, 0 |
| 5A | 1,120 | 114 | 29 | 4,4 3 | 0,73 | 10,2 | 128 | 1,036 | 166 | 4,6 1 | 1,00 | 16, 4 |

In this case, it can also be seen that the degree of distilled alcohol is substantially higher for the CECT 13152 strain than for the commercial strain under all the conditions tested.

Table 5 lists some of the parameters that demonstrate the higher fermentation and alcohol production capacity of the strain of the invention:

**Table 5**

| **CODE** | **1A** | **1B** | **2A** | **2B** | **3A** | **3B** | **4A** | **4B** | **5A** | **5B** | **UNITS** |
|---|---|---|---|---|---|---|---|---|---|---|---|
| **η _{THEORETICAL}** | 0.5 11 | 0.5 11 | 0.5 11 | 0.5 11 | 0.5 11 | 0.5 11 | 0. 51 1 | 0.5 11 | 0.5 11 | 0.5 11 | g EtOH/g glucose |
| **DISTILLED ALCOHOL CONTENT** | 13 | 15. 1 | 10. 4 | 12 | 9.5 | 9.7 | 3. 8 | 9.2 | 10. 2 | 12 | °GL |
| | 10 2.6 5 | 11 9.2 3 | 82. 12 | 94. 75 | 75. 01 | 76. 59 | 30 .0 0 | 72. 64 | 80. 54 | 94. 75 | g Pure EtOH/L |
| **FERMENT SUGAR OVER ALCOHOL** | 20 0.8 3 | 23 3.2 8 | 16 0.6 7 | 18 5.3 8 | 14 6.7 6 | 14 9.8 5 | 58 .7 1 | 14 2.1 3 | 15 7.5 8 | 18 5.3 8 | 9 fermentable sugars/L |
| **FERMENTABLE SUGARS MEASURED HPLC** | 25 0 | 25 0 | 20 0 | 20 0 | 17 2 | 17 2 | 25 0 | 25 0 | 20 0 | 20 0 | g/L |
| **SUBSTRATE CONCENTRATION** | 25 0 | 25 0 | 20 0 | 20 0 | 17 2 | 17 2 | 25 0 | 25 0 | 20 0 | 20 0 | g/L |

| **% FERMENTABLE SUGARS IN THE ORIGINAL SUBSTRATE (WITHOUT DILUTION)** | | | | | | | | | | | |
|---|---|---|---|---|---|---|---|---|---|---|---|
| **THEORETICAL (WITH RESPECT TO °GL OBTAINED)** | 80 | 93 | 80 | 93 | 85 | 87 | 23 | 57 | 79 | 93 | % |
| **ACTUAL (WITH RESPECT TO MEASURED SUGARS)** | 10 0 | 10 0 | 10 0 | 10 0 | 10 0 | 10 0 | 10 0 | 10 0 | 10 0 | 10 0 | % |
| **η _{MAXIMUM ETHANOL}** | 52 0 | 60 4 | 52 0 | 60 0 | 55 2 | 56 4 | 15 2 | 36 8 | 51 0 | 60 0 | L.A.P./ Tm substrate |

### Example 3: Fermentation capacity of the mutant Saccharomyces cerevisiae CECT 13152.

The fermentative capacity of a new mutant strain of *Saccharomyces cerevisiae* CECT 13152 was determined by fermentation from starch substrates obtained from tubers, cereal hydrolysates and fruit wastes.

### (a) Starch substrate hydrolysis

As the sugars present in the starchy substrates used are in the form of starch and the yeast used lacks amylases, it was necessary to carry out prior hydrolysis of these substrates. In addition, the substrate was liquefied once it was mixed with water.

### Mixture of starch-water substrate

The starch substrates used, i.e. grain, tubers and fruit wastes were crushed and sieved with a diameter of approximately 1mm and mixed with water at a ratio of 38:62 (substrate: water) (w/w).

### Liquefaction in 2 stages

Stage 1. 20% of the total dose of α-amylase (Liquozyme, 240 KNU/g) was added to each of the mixtures and incubated for a period of 45 minutes at 65°C under constant stirring, intense enough to keep the substrate suspended.

Stage 2. The remaining 80% of α-amylase (Liquozyme, 240 KNU/g) was added to the mixture and incubated for 30 minutes at 85°C under constant stirring as in stage 1.

### Autoclave

The processed starch compounds were placed in the autoclave and subjected to a temperature of 130°C for 3 minutes.

### Saccharification

The dose of amyloglucosidase (Spirizyme Fuel) was added and incubated at 65° C, for 90 minutes maintaining constant stirring, the same as in previous stages.

Dilutions were made from the different substrates, to obtain a sugar concentration of 27%.

Nutrients were added to the fermentation media where necessary.

### (b) Yeast and propagation conditions

The mutant yeast *Saccharomyces cerevisiae* CECT 13152 was used to carry out the fermentation. This was done by propagation prior to fermentation. Yeast propagation was carried out in two stages: an initial stage in YPD and a second stage in a sugar substrate of the same nature as the one used in fermentation, at a sugar concentration of between 7-8%. The propagation temperature was 34.5°C, stirring was at 120 rpm and pH was 5. Depending on the type of substrate, the addition of salts and vitamins was necessary. The use of aeration during this second stage considerably increased the number of cells obtained per mL at the end of the process.

### (c) Fermentation conditions

Fermentation took place at a constant temperature of 35°C. As for the stirring, the test was started without stirring, and remained so for the first hour. The second hour was programmed at 40 rpm. Finally, 85 rpm were set at the beginning of the third hour, and this was maintained until the end of fermentation. The end of fermentation was defined as the moment when the loss of CO₂ between two consecutive weight measurements is zero, or when exceeding 100 hours of fermentation.

The results of the fermentation tests on the different substrates are shown in tables 6 to 8 below.

**Table 6. Initial sugars (% w/v).**

| Tubers | Cereal hydrolysates | Fruit waste |
|---|---|---|
| 27 | 27 | 27 |

**Table 7. Alcohol concentration obtained (°G.L.)**

| Tubers | Cereal hydrolysates | Fruit waste |
|---|---|---|
| 16,1° | 16,2° | 16,2° |

**Table 8. Final sugars (% w/v).**

| Tubers | Cereal hydrolysates | Fruit waste |
|---|---|---|
| 0,2 | < 0,1 | < 0,1 |

## Claims

1. Strain of the species *Saccharomyces cerevisiae* deposited in the Spanish Type Culture Collection (CECT) with access number CECT 13152.

2. Strain according to claim 1 **characterized in that** it is in liquid, fresh paste, active dry or instant dry format.

3. Use of the strain of claim 1 or 2, in the production of ethanol or the manufacture of alcoholic products by fermentation.

4. Use according to claim 3, wherein ethanol is produced by alcoholic fermentation of plant residues and lignocellulosic residues.

5. Use according to claim 3, wherein the alcoholic product is an alcoholic beverage.

6. Use according to claim 5, wherein the alcoholic beverage is wine, beer, champagne, cider or distilled beverages.

7. Use according to claim 3, wherein alcoholic fermentation produces ethanol at 16° G.L. or higher or other alcoholic products.

8. Use of the strain according to any of claims 1 or 2 for baking.

9. Use of a strain according to any of claims 1 or 2 for biomass production.

10. Use according to claim 9, wherein the biomass has application as a source of animal feed.

11. Microbiological composition comprising a strain according to any of claims 1 and 2 and optionally at least one additional element that may benefit the production of alcohols or alcoholic products and/or the fermentation process.

12. Composition according to Claim 11 in the form of diluted aqueous, cream, pressed, dried or freeze-dried composition.

## Patentansprüche

1. Stamm der Art Saccharomyces cerevisiae, hinterlegt in der Spanish Type Culture Collection (CECT) unter der Zugangsnummer CECT 13152.

2. Stamm nach Anspruch 1, **dadurch gekennzeichnet, dass** er in flüssiger, frischer Paste, aktivem trockenem oder sofortigem trockenem Format vorliegt.

3. Verwendung des Stammes nach Anspruch 1 oder 2 bei der Produktion von Ethanol oder der Herstellung von alkoholischen Produkten durch Gärung.

4. Verwendung nach Anspruch 3, wobei Ethanol durch alkoholische Gärung von Pflanzenrückständen und lignocellulose-Rückständen hergestellt wird.

5. Verwendung nach Anspruch 3, wobei das alkoholische Produkt ein alkoholisches Getränk ist.

6. Verwendung nach Anspruch 5, wobei das alkoholische Getränk Wein, Bier, Champagner, Apfelwein oder destillierte Getränke ist.

7. Verwendung nach Anspruch 3, wobei durch die alkoholische Gärung Ethanol mit 16° G.L. oder mehr oder andere alkoholische Produkte erzeugt werden.

8. Verwendung des Stammes nach einem der Ansprüche 1 oder 2 zum Backen.

9. Verwendung eines Stammes nach einem der Ansprüche 1 oder 2 zur Erzeugung von Biomasse.

10. Verwendung nach Anspruch 9, wobei die Biomasse als Tierfutterquelle verwendet wird.

11. Mikrobiologische Zusammensetzung, die einen Stamm nach einem der Ansprüche 1 und 2 und gegebenenfalls mindestens ein zusätzliches Element enthält, das die Herstellung von Alkoholen oder alkoholischen Produkten und/oder den Fermentationsprozess begünstigen kann.

12. Zusammensetzung nach Anspruch 11 in Form einer verdünnten wässrigen, cremigen, gepressten, getrockneten oder gefriergetrockneten Zusammensetzung.

## Revendications

1. Souche de l'espèce Saccharomyces cerevisiae déposée dans la Collection espagnole de cultures types (CECT) avec le numéro d'accès CECT 13152.

2. Souche selon la revendication 1 **caractérisée par le fait qu'**elle se présente sous forme liquide, pâte fraîche, sèche active ou sèche instantanée.

3. Utilisation de la souche de la revendication 1 ou 2, dans la production d'éthanol ou la fabrication de produits alcoolisés par fermentation.

4. Utilisation selon la revendication 3, dans laquelle l'éthanol est produit par fermentation alcoolique de résidus végétaux et de résidus ignocellulosiques.

5. Utilisation selon la revendication 3, dans laquelle le produit alcoolisé est une boisson alcoolisée.

6. Utilisation selon la revendication 5, dans laquelle la boisson alcoolisée est du vin, de la bière, du champagne, du cidre ou des boissons distillées.

7. Utilisation selon la revendication 3, dans laquelle la fermentation alcoolique produit de l'éthanol à 16° G.L. ou plus ou d'autres produits alcooliques.

8. Utilisation de la souche selon l'une des revendications 1 ou 2 pour la cuisson.

9. Utilisation d'une souche selon l'une des revendications 1 ou 2 pour la production de biomasse.

10. Utilisation selon la revendication 9, dans laquelle la biomasse est utilisée comme source d'alimentation animale.

11. Composition microbiologique comprenant une souche selon l'une des revendications 1 et 2 et éventuellement au moins un élément supplémentaire pouvant favoriser la production d'alcools ou de produits alcooliques et/ou le processus de fermentation.

12. Composition selon la revendication 11 sous forme de composition aqueuse diluée, de crème, pressée, séchée ou lyophilisée.
